# EUROPEAN PATENT SPECIFICATION

(11) **EP 4 488 042 B1**
(45) Date of publication and mention of the grant of the patent: **14.10.2026**
(21) Application number: 23183732.9
(22) Date of filing: 05.07.2023
(51) Int. Cl.: B29C 65/14, B29C 65/20, B29C 65/68, B29C 65/78, A61M 39/14, A61M 39/16, B29L 23/00, B29K 83/00

(54) **STERILE CONNECTION**
STERILE VERBINDUNG
CONNEXION STÉRILE

(43) Date of publication of application: 08.01.2025
(73) Proprietor: Sartorius Stedim FMT, 13400 Aubagne (FR)
(72) Inventor: Mueller, Carsten, 13600 La Ciotat (FR); Montenay, Nelly, 13790 Rousset (FR); Delaunay, Lucie, 13112 La Destrousse (FR); Bieber, Lola, 13006 Marseille (FR)
(74) Representative: Novagraaf International SA

(56) References cited:
- EP-A1- 0 507 321
- WO-A1-2024/171077
- WO-A1-2024/256449
- US-A1- 2017 252 550
- US-A1- 2021 001 566
- US-A1- 2022 134 461

## Description

### Field of the invention

The invention relates to a method for connecting a cut end of a silicone tube with a connecting element and a device to implement such method.

### Background

Plastic tubes, generally having a circular cross-section, make circulation, passage and communication of a fluid possible. Many fields, such as biotechnology, chemistry, medical industry and pharmaceutical industry, use sterile connections from plastic tubes for the delivery and removal of fluids or for sealing a fluid path.

Most often, the operations to which such tubes are subjected-and in particular cutting them into sections-must result in an aseptic way, i.e., the bacterial integrity of the tubes must be guaranteed.

In some cases, a tube that channels fluid needs to be cut into sections. To this aim, circulation of the fluid through a given tube region needs to be stopped by closing the tube at the given region making it possible to separate the tube into two sections or portions. The cut sections are then either sealed or connected to other tube sections (such as described in document US8857485), optionally with a particular function such as storing, filtering, etc.

Sealing and connecting the cut sections are usually performed in a non-sterile environment and thus usually involve heating a cutting member like a cutting blade. The heated cutting blade is then contacted with the tube thereby partially melting it allowing it to fuse or seal with a stop or another tube sections.

However, as opposed to commonly used thermoplastic tubes, silicone tubes are more difficult to handle.

Silicones are preferred for their outstanding and unique mechanical properties and inertness. While silicone is a preferred material for fluid circulation due to its advantageous flexibility and pump characteristics, , silicone softens when heated; making it difficult to cut with a heated cutting member. Furthermore, silicone is sticky once heated making it difficult for two portions of a silicone tube to be cleanly severed from each other.

As a result, cutting a silicone tube with the aim to connect it to another fluid path or to seal it in a sterile way remains a challenge.

It is therefore an aim of the present application to provide a method that results in an aseptic connection or sealing of silicone tubes.

US2022134461 A1 discloses forming a sterile connection between two polymeric profiles, accomplished by coincidentally welding the ends of the profiles via a surface activation treatment. Also disclosed is an irradiation welding apparatus including a cutting device.

US2021001566 A1 discloses forming a sterile connection between two silicone elastomer profiles, by coincidentally welding the ends of the profiles via a surface activation treatment.

US2017252550 A1 discloses a device for connecting tubing, the device having driven carriages for holding and moving connectors of tubing towards a coupler, and a UV-C light source for sterilising.

### Summary

The present invention provides a method for sterilely connecting a cut end of a silicone tube with a connecting element as defined in claim 1.

The present invention also provides a device comprising an operation chamber as defined in claim 12.

Other optional and non-limiting features are as follows.

The method may further comprise sterilising the connecting element using the UVC radiation thereby obtaining a sterilised connecting element prior to connecting.

The UVC radiation may be a radiation having one or more wavelengths of 100 nm to 280 nm, thus may comprise or fully consist of VUV radiation.

The VUV radiation may have one or more wavelengths of 100 nm to 200 nm, preferably in the range between 150nm and 190nm, preferably around 172 nm.

The advantage of this method is that it provides an aseptic connection in any environment. Thus, a cleanroom environment is not necessary. This is particularly important since it leads to less constraining requirements than cleanroom environment.

The method is advantageously carried out in a device with controlled parameters, selected among the following list: temperature, humidity.

The method may comprise sterilising the environment of the silicone tube and connecting element.

Sterilising the cut end and sterilising the connecting element may be performed using same VUV wavelength or different VUV wavelengths.

This is particularly advantageous for a silicone tube made of silicone since sterilising under VUV radiation would provide the further effect of activating the surface of the silicone, thus promoting adherence of the cut-end of the silicone tube to the connecting element.

The activation of the surface of the silicone leads to a modified surface thereof. The modification may include a structural change and/or a change in the material composition of the modified surface compared to the unmodified silicone. The modification particularly takes place by means of VUV radiation having a wavelength of 200 nm or lower.

VUV radiation on the cross-section of the cut end of the silicone tube brings further structural modifications thereon, leading to the emergence of cracks.

It has been shown that the modification of the silicone surface according to the invention leads, at least in the preferred cases, to a slight increase in the oxygen content and only a slight reduction in the carbon content compared to the unmodified silicone, as indicated in WO 2015/075040. In this context, it is assumed that the reduced carbon content is caused by the degradation of carbon functions (here caused by radiation), accompanied by the incorporation of oxygen functions, in particular the formation of silanol groups.

Consequently, the sterilised cut end presents an activated surface which is prone to connect to a sterile connecting element, thereby providing a sterile adhesiveless connection, without adding any bonding material or additive.

Furthermore, also in case of silicone, the modification of the silicone surface leads to the emergence of crack structures with a depth of 2-50 µm and thus of a rough area, like it has been shown in WO 2016/030183.

Such method ensures the obtention of a physically resistant, sterile adhesiveless connection between a sterilised cut end of a silicone tube with a connecting element without any added bonding material. Indeed, the connection between the VUV-treated cut end and connecting element is carried out immediately or very shortly after the sterilising step.

The mechanical robustness of the weld that can be achieved does exceeds the mechanical material robustness.

The silicone tube may be opaque to UVC radiation.

The connecting element may be made of silicone.

Connecting may comprise contacting the cut end with the connecting element. Indeed, connecting may further comprise placing in direct contact the silicone tube cut end and the connecting element and coincidentally bonding them together, thereby providing a sterile adhesiveless connection.

The method may further comprise a cutting step before sterilising the cut end and sterilising the connecting element, wherein the cutting sterilising a cutting member and transversely cutting a silicone tube with the cutting member, thereby providing two silicone tube portions having each a cut end. Advantageously, the cutting member is at room temperature during cutting.

According to an alternative embodiment, the cutting member is at a temperature above the room temperature during cutting, preferably at a temperature lower than 150°C.

According to an alternative embodiment, the cutting member is at a temperature below the room temperature during cutting.

Sterilising a cutting member is preferably performed using UVC radiation. However, it may alternatively be performed by heating.

The UVC radiation may typically be a radiation of lower than 280 nm (known as UVC).

The UVC radiation may comprise at least partially VUV radiation.

The method may further comprise a clamping step before cutting, wherein the clamping comprises clamping the silicone tube at a clamping site thereof, thereby obtaining a sealed region within the tube, and wherein transversely cutting is subsequently performed at a cutting site adjacent the clamping site, thereby providing the cut end. Clamping the silicone tube may also comprise clamping the silicone tube at a further clamping site, wherein the cutting site is located between the clamping site and the further clamping site.

Clamping the silicone tube and transversely cutting it at its cutting site may be performed concomitantly.

The method may further comprise another sterilising step of the silicone tube and/or the connecting element using UVC radiation prior to its clamping and/or cutting.

The method may further comprise sterilising the silicone tube and the connecting element concomitantly using UVC radiation prior to the clamping or cutting of the silicone tube.

The distance between the clamping site and the cutting site and the distance between the further clamping site and the cutting site are preferably chosen so that a flattened zone is obtained between the clamping site and the further clamping site. Transversely cutting is thus carried out in the flattened zone, such as described in document US8857485.

Clamping the silicone tube may comprise clamping the silicone tube at a first clamping site and a second clamping site, wherein the second clamping site is between the first clamping site and the cutting site, and the method may further comprise unclamping the tube at the second clamping site, thereby providing an unclamped cut end. Clamping the silicone tube may also comprise before transversely clamping the silicone tube at a first further clamping site and a second further clamping site before transversely cutting, wherein the second further clamping site is located between the first further clamping site and the cutting site, and wherein the cutting site is located between the second clamping site and the second further clamping site. The distances between:
- the first clamping site and the second clamping site;
- the second clamping site and the cutting site;
- the cutting site and the second further clamping site; and
- the second further clamping site and first further clamping site;
are preferably chosen so that a flattened zone is obtained between the first clamping site and the first further clamping site.

However, the distances between:
- the first clamping site and the cutting site; and
- the first further clamping site and the cutting site;
are preferably chosen so that when the second clamping site and the second further clamping site are unclamped following transversely cutting, the cut ends are in an unclamped open state.

Alternatively to clamping the tube at a second clamping site and at a second further clamping site, unclamping the tube cut ends may be achieved through moving the first clamping site and the first further clamping site further away from the tube cut ends.

Connecting the sterilised cut end of a silicone tube with the connecting element may comprise unclamping the silicone tube portion.

The method may further comprise sterilising the adhesiveless connected assembly UVC radiation after the unclamping of the silicone tube portion.

Between the wavelengths of around 200 and around 280 nanometers (nm), which corresponds to UVC domain, nucleic acids in a microbe are disrupted. This leads to cell death in bacteria and inactivation in viruses.

UVC radiation of 280 or lower possesses excellent germicidal properties to inactivate a wide range of microbial pathogens (e.g., bacteria, fungi, yeasts, molds, and viruses). In particular, between about 200 nm and about 280 nm, which is where UV-C operates, nucleic acids in a microbe are disrupted. This leads to cell death in bacteria and inactivation in viruses.

In a sealing embodiment, the connecting element may be a sealing element, for example a stop or a heat shrinkable sleeve. The sealing element may be made of silicone.

The stop may be a slice of material with a cross section contacting and covering a cross section of the cut end.

Alternatively, the stop may be a rod with a cross section contacting or covering a cross section of the cut end.

In this latter case, the method may comprise clamping the silicone tube at a clamping site thereof and a further clamping site thereof, the cutting site being positioned between the clamping site and the further clamping site; transversely cutting the silicone tube at a cutting site between the clamping site and the further clamping site using the cutting member, thereby providing two tube portions each having a cut end; relatively moving the cutting member away from the cutting site; transversely cutting the rod using the cutting member, thereby providing two rod portions, each having a rod cut end; and contacting each rod cut end with a corresponding tube cut end.

After contacting, the cross sections of the tube cut end may be kept in a clamped state. A heat shrinkable sleeve may also be added, as described below.

Transversely cutting the tube and transversely cutting the rod may be performed with a single relative movement of the cutting member.

The method may further comprise before transversely cutting the tube, disposing the tube and the rod so that their longitudinal axes are colinear with each other, thereby transversely cutting provides two sets, each having a tube portion and a rod portion; and rotating one set relatively to the other set by 180° and around a rotation axis which is colinear with the longitudinal axes of the tube portions and the rod portions.

The plane containing both longitudinal axes may have any orientation. In particular, the plane may be horizontal, vertical or angled at for example 45° relative to a vertical line.

When the stop is a heat shrinkable sleeve, connecting may comprise introducing the sterilised cut end through an open end of the heat shrinkable sleeve and heating the heat shrinkable sleeve, thereby providing an adhesiveless seal at the cut end. Sterilising may be performed after or during connecting.

In a welding embodiment, the silicone tube may be welded to another second silicone tube. In such embodiment, the connecting element may be the second silicone tube itself. A heat shrinkable sleeve may also be added, as described below.

When the connecting element is a second silicone tube, the method may comprise clamping the silicone tube at a clamping site thereof and a further clamping site thereof, the cutting site being positioned between the clamping site and the further clamping site; transversely cutting the silicone tube at a cutting site adjacent the clamping site and the further clamping site using a cutting member, preferably unheated, thereby providing two tube portions each having a cut end; clamping the second silicone tube at a clamping site thereof, thereby obtaining a sealed region within the second silicone tube; transversely cutting the second tube at a cutting site adjacent the clamping site using the cutting member, thereby two tube portions each having a cut end; and contacting each cut end of the silicone tube with a corresponding cut end of the second silicone tube portions.

Clamping the second silicone tube may be performed at the clamping site and a further clamping site thereof, the cutting site being positioned between the clamping site and the further clamping site.

Transversely cutting the silicone tube and transversely cutting the second silicone tube may be performed with a single relative movement of the cutting member.

The method may further comprise before transversely cutting the silicone tube and the second silicone tube, disposing the silicone tube and the second silicone tube so that their longitudinal axes are colinear with each other, thereby transversely cutting provides two sets, each having a tube portion of the silicone tube and a tube portion of the second silicone tube; and rotating one set relatively to the other set by 180° and around a rotation axis which is colinear with the longitudinal axes of the tube portions.

The plane containing both longitudinal axes may have any orientation. In particular, the plane may be horizontal, vertical or angled at for example 45° relative to a vertical line.

When the connecting element is a heat shrinkable sleeve, connecting may comprise introducing the cut end through one end of the heat shrinkable sleeve, introducing a cut end of a second silicone tube through the other end of the heat shrinkable sleeve and heating the heat shrinkable sleeve, thereby providing an adhesiveless seal at the cut ends of the silicone tube and the second silicone tube. Sterilising may be performed after or during connecting.

In all embodiment, the method may comprise at the end unclamping all (remaining) clamping sites.

### Brief description of the drawings

The present disclosure may be better understood and its numerous features and advantages made apparent to those skilled in the art by referencing and accompanying drawings.
Fig. 1A-1C show diagrams illustrating an exemplary silicone tube that is cut, sterilised and connected to a connecting element according to the present disclosure.
Fig. 2A-2C include illustrations of two clamped states of the cut end and an unclamped open state of the cut end.
Fig. 3A-3G include illustrations of a sealing embodiment.
Fig. 4A-4G include illustrations of a sealing embodiment.
Fig. 5A-5H include illustrations of a welding embodiment.
Fig. 6A-6I include illustrations of a welding embodiment.
Fig. 7A-7C include illustrations of a device according to an embodiment.

### Detailed description

The use of the same reference symbols in different drawings indicates similar or identical items.

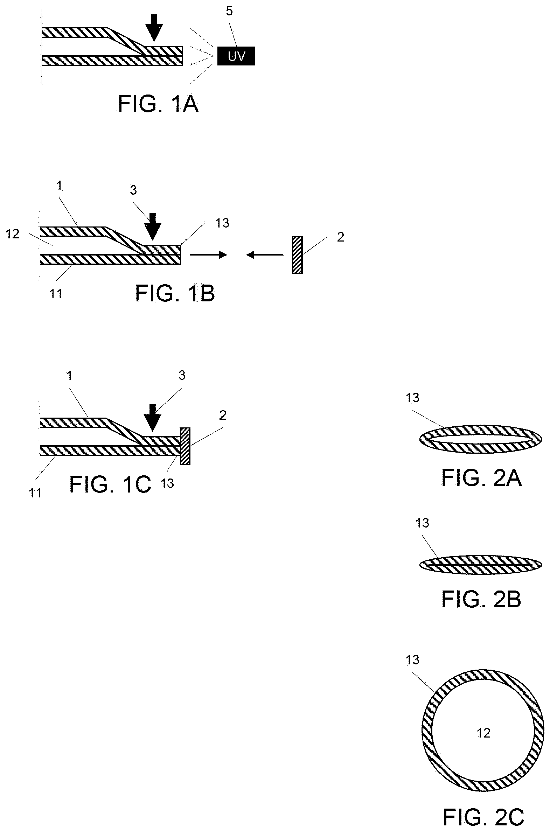

Hereinafter a method for providing a sterilised region at a cut end of a silicone tube will be described with reference to Fig. 1A-1C.

The silicone tube is any tube which is commercially available or tailored to a particular need. Its cross-section is typically circular but the method is not limited to such geometry of tube. For example, the cross-section may have any other shape such as rectangular (including squared), triangular, pentagonal, hexagonal, octagonal, etc. The cross-section may also have more complex shape and needs not be polygonal, i.e. it may comprise curved portions or being fully curved.

The method comprises sterilising the cut end **13** of the silicone tube **1** using a UVC radiation **5** (Fig. 1A) and connecting it with a connecting element **2** (Fig. 1B) thereby providing an adhesiveless connected assembly **4** (Fig. 1C).

The method may further comprise sterilising the cut end and the connecting element using more specifically VUV (vacuum ultraviolet) radiation , thereby obtaining a sterile tube cut end and a sterile connecting element.

In this method, sterilisation of the connected assembly is carried out using VUV radiation **5.** The combination of the VUV radiation and the silicone nature of the tube and the connecting element makes it possible not only to sterilise the outer surface of the tube and connecting element, but also spaces contained in between the tube and connecting element as well as spaces within the tube in the vicinity of the cut end (typically the outer surface of the VUV lamp lies within 1 mm to 2 cm from the cut end, preferably between 2 mm to 1 cm, or 3 mm to 7 mm, for example about 5 mm).

Sterilising the cut end and sterilising the connecting element may be performed concomitantly.

Typically, the silicone tube is any commercially available tube that is opaque to UV radiations. The silicone tube **1** has a longitudinal axis and presents a central lumen **12** extending along its longitudinal axis. The central lumen **12** serves as a fluid pathway for a fluid to flow therethrough.

The cut end **13** may be previously obtained through cutting out a section of a silicone tube 1, for example with a cutting blade.

Preferably, the connecting element is made of the same material as the silicone tube.

Silicone is an elastomer which includes a silicone matrix component. An exemplary silicone matrix component includes a polyorganosiloxane. An exemplary polyorganosiloxane includes a polyalkylsiloxane, a polyarylsiloxane, or combination thereof. Any reasonable polyalkylsiloxane is envisioned. Polyalkylsiloxanes include, for example, silicone polymers formed of a precursor, such as dimethylsiloxane, diethylsiloxane, dipropylsiloxane, methylethylsiloxane, methylpropylsiloxane, or combinations thereof. In a particular embodiment, the polyalkylsiloxane includes a polydialkylsiloxane, such as polydimethylsiloxane (PDMS). In a particular embodiment, the polyalkylsiloxane is a silicone hydride-containing polyalkylsiloxane, such as a silicone hydride-containing polydimethylsiloxane. In a further embodiment, the polyalkylsiloxane is a vinyl-containing polyalkylsiloxane, such as a vinyl-containing polydimethylsiloxane. The vinyl group may be an endblock of the polyalkylsiloxane, on cof theofthe polyalkylsiloxane, or any combination thereof. In yet another embodiment, the silicone matrix component is a combination of a hydride-containing polyalkylsiloxane and a vinyl-containing polyalkylsiloxane.

The method is advantageously carried out in a controlled environment. A controlled environment refers to a specially designed and regulated setting where specific parameters are manipulated, monitored, and maintained to facilitate the implementation and execution of the method. This environment is carefully controlled to ensure consistency, accuracy, and reproducibility of the method's outcomes. In such a controlled environment, various parameters such as humidity, temperature, or any other relevant parameter may be regulated to optimize the outcome of the method. In particular, the method comprises controlling the humidity of the environment. The method may further comprise controlling the temperature of the environment.

Connecting may comprise contacting the cut end **13** with the connecting element **2.**

Before connecting the cut end **13** with the connecting element **2,** the method may comprise sterilising a cutting member and transversely cutting a silicone tube with the cutting member, wherein the cutting member is at room temperature during cutting.

A cutting member refers to a tool specifically designed and employed for the purpose of cutting or severing the material making up the silicone tube. It is typically a sharp object that exhibits cutting edge or edges capable of penetrating and dividing the silicone tube through the action of pressure and/or motion.

Any method of cutting is envisioned. Its purpose is that the cutting of the tube can be done from one side to the other. As appropriate, the tube is cut into sections by means of a single cutting element or two opposite cutting elements. Examples of cutting members include blades, knives, scissors, shears, rotary cutters, scalpels, or any other tool or instrument engineered to perform cutting

Before cutting, the method may comprise clamping the silicone tube at a clamping site thereof, thereby obtaining a sealed region within the tube, and wherein transversely cutting is performed at a cutting site adjacent the clamping site, thereby providing silicone tube portions each present a cut end.

Fig. **2A** and **2B** illustrate the state of the cut end **13** of the silicone tube when clamped. Depending on the distance between the clamping site **31** and the cut end **13,** this latter can be relatively closed and slightly open (Fig. **2A****)** or completely closed (Fig. **2B).** Fig. **2C** illustrates the unclamped state of the cute end **13.**

A tube-flattener **3** ensures that the fluid circulating within the central lumen **12** is stopped at the so created flattened region or a sealed region. The newly formed sealed region prevents the circulation, the passage, and the communication of fluid on either side of this region.

Following transversely cutting, the cut end is in a clamped state illustrated by Fig. **2A** or **2B** in such a state, the overall cross section of the cut end is reduced so that opposite portions of the tube wall are brought closer towards each other, sometimes even contacting each other.

Clamping the silicone tube may be advantageous when the method is carried out while fluid flows through the silicone tube. The fluid may be any flowable material and includes, but is not limited to, liquid, gas, solid particles, or combination thereof such as slurry. Clamping makes it possible to stop the fluid flow before transversely cutting. Accordingly, the silicone tube is compressed and flattened at its clamping site under mechanical pression thereby forming a sealed region. As a result, the fluid flow passing though the silicone tube is stopped prior to the sealed region and the fluid cannot go beyond the sealed region. Thus, the fluid is stopped prior to the cutting step and cannot reach the cutting site. This also allows maintaining the sterility in the inside of the silicone tube while cutting.

In such case, a cutting site is designated and two clamping sites on both sides thereof, adjacent to the cutting site, are chosen. A cutting member cuts the silicone tube at the cutting site. Two tube portions are thus obtained, each having a tube cut end.

Clamping the silicone tube may comprise clamping the silicone tube at a first clamping site and a second clamping site, wherein the second clamping site is between the first clamping site and the cutting site, and the method may further comprise unclamping the tube at the second clamping site, thereby providing an unclamped open cut end **13** as illustrated in Fig. **2C****,** presenting the central lumen **12.**

The use of two clamping sites and unclamping the second clamping site is particularly advantageous when it is desired to connect the cut end of the tube with the connecting element in an unclamped state while avoiding contact of the remaining portion of the tube with the exterior environment.

However, instead of clamping the tube at a second clamping site, the unclamped state of the cut end may also be achieved through moving the first clamping site further away from the cut end to allow this latter to reopen. This can be obtained through use of compressing rollers with longitudinal axes perpendicular to the longitudinal axis of the tube.

The cut end of the silicone tube may be sterilised both before and after unclamping the second clamping site.

If connecting the cut end of the tube with the connecting element in an unclamped state is desired, the sterilised silicone tube cut end and the silicone connecting element are placed in direct contact so that either the cross-section of the silicone tube cut end or the cross-section of the silicone connecting element be covered and engaged to the other one, thereby providing a sterile adhesiveless connection.

Clamping can be carried out with any tube-flattener **3,** such as clamp(s), screw(s), tie(s), or combination thereof. When the silicone tube or the connecting element comprises various clamping sites, clamping may be carried out with at least one tube-flattener at each clamping site. When clamping jaws are employed, the clamping arrangement might contain multiple sub clamps, each configured to seal a silicone tube or a connecting element at multiple clamping sites, either adjacent to each other or distant.

Typically, a compression force of 25 to 500 N is applied during clamping.

Sterilising the cut end may be performed through a UVC-protection screen to limit radiation of the silicone tube to the cut end. The UV-protection screen may typically be made of a UVC-opaque material with cut out(s) to be placed facing the cut end. Likewise, sterilising the connecting element may be performed through a UVC-protection screen. In such case, the cut out(s) is to be facing the connecting element during sterilising.

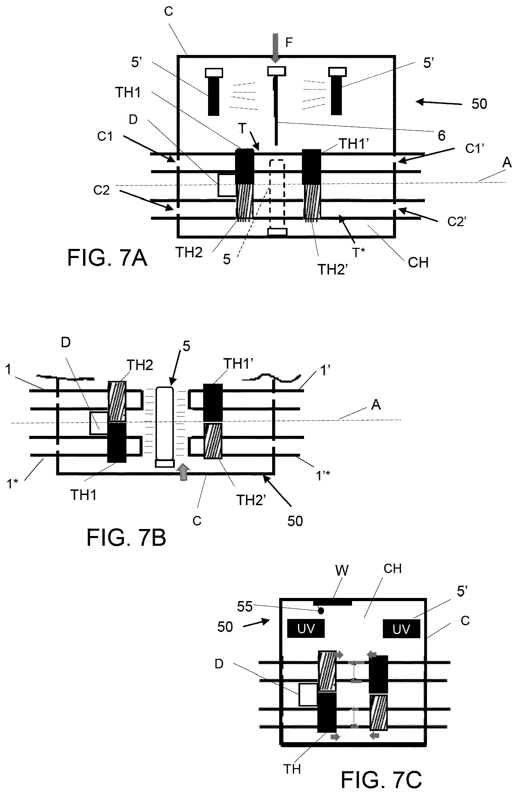

The method may be implemented in a device **50** which will now be described with reference to Fig. 7A to 7C.

The device comprises an operation chamber **CH,** and within the operation chamber **CH** a UVC radiation source **5,** a tube support **TH1, TH1'** and an additional support **TH2, TH2'** for the connecting element **2.** The tube support **TH1, TH1'** is configured to hold the silicone tube **T** and the additional support **TH2, TH2'** is configured to hold the connecting element **2.** The device **50** also comprises a driver **D** configured to move the tube support **TH1, TH1'** and the additional support **TH2, TH2'** relative to each other.

The UVC radiation source **5** is configured to emit a radiation comprising one or more wavelengths from 100 nm to 280 nm. Preferably, the UVC radiation source **5** is configured to emit a radiation comprising at least partially VUV radiation or consists totally of VUV radiation. Alternatively, a UVC radiation source **5'** and a VUV radiation source **5** are both provided. An example of such VUV radiation source **5** is a stable Excimer light source (for instance involving xenon). A continuous-wave light source at 172 nm is preferred.

The tube support **TH1, TH1'** typically defines a support direction corresponding to the direction of the longitudinal axis of the tube **T** once received on the tube support **TH1, TH1'.** The tube support **TH1, TH1'** may comprise a tube flattener configured to clamp a tube at a clamping site **31** and a further clamping site **31'** and provide a sealed region between the clamping site **31** and the further clamping site **31'.** The tube flattener may be a set of clamps or jaws. The set of clamps or jaws may be alternatively in the form of rollers with axes perpendicular to the axis of the tube and movable along the axis of the tube.

The set of clamps or jaws may alternatively be configured to clamp a tube at a clamping site **31,** a second clamping site **32,** a second further clamping site **32'** and a further clamping site **31',** in this order. In this latter case, the set of clamps or jaws are preferably configured to clamp the clamping site **31** and the further clamping site **31'** independently from the second clamping site **32** and further second clamping site **32'.**

The additional support **TH2, TH2'** may be configured to hold a slice of material **21,** a rod **R,** a second silicone tube **T*** or a heat shrinkable sleeve **8.**

When it is configured to hold a rod **R,** the additional support **TH2, TH2'** typically defines an additional support direction corresponding to the direction of the longitudinal axis of the rod **R** once this latter is received on the additional support **TH2, TH2'.** The additional support **TH2, TH2'** may comprise a set of holders configured to hold the rod **R** at two locations thereof.

When it is configured to hold a second silicone tube **T*,** the additional support **TH2, TH2'** typically defines an additional support direction corresponding to the direction of the longitudinal axis of the second tube **T*** once this latter is received on the additional support **TH2, TH2'.** The additional support **TH2, TH2'** may be configured in the same manner as the tube support **TH1, TH1'** described above.

The tube support **TH1, TH1'** and the additional support **TH2, TH2'** may be arranged next to each other and in particular so that the support direction and the additional support direction are colinear with each other.

The driver **D** is configured to bring the held tube **T** and connecting element **2** in contact with each other.

The device **50** may further comprise a movable cutting member **6.** The movable cutting member **6** is configured to move from a rest position to a cutting position and along a cutting direction. The UVC radiation source **5** may be configured to emit a UVC radiation towards the movable cutting member **6,** in particular while in its rest position.

The cutting direction is configured to pass through the silicone tube **T** when this latter is received by the tube support **TH1, TH1',** notably between the clamping site **31** and the further clamping site **31'** or between the second clamping site **32** and the second further clamping site **32'** when these are provided. The course of the movable cutting member **6** is sufficiently extended so that the movable cutting member **6** can cut a silicone tube **T** received by the tube support **TH1, TH1'** and a connecting element **2** received by the additional support **TH2, TH2',** such as a rod **R** or a second tube **T*,** in one movement.

The cutting direction thus divides each of the operation chamber **CH,** the tube support **TH1, TH1'** and the additional support **TH2, TH2'** into two halves. In such case, on each side of the cutting direction, there is a set comprising a portion of the tube support **TH1, TH1'** and a portion of the additional support **TH2, TH2'.** The driver **D** may be configured to rotate one set relatively to the other by 180° and about a direct that is collinear with the support direction and the additional support direction. In such a configuration, the portions of the tube support **TH1, TH1'** and the portions of the additional support **TH2, TH2'** are interchangeable. This means that they play the role of a tube support **TH1, TH1'** or an additional support **TH2, TH2'** depending on their position determined by the driver **D.**

The device **50** may comprise a controller for controlling the environment of the operation chamber **CH,** for example humidity and/or temperature.

The device **50** may comprise a casing **C** forming the operation chamber **CH.** The tube support **TH1, TH1'** and the additional support **TH2, TH2'** are housed inside the casing **C.** The cutting member **6** may be provided so that its resting position and its cutting position are both inside the casing **C** or only its cutting position is inside the casing **C.** The resting position can also be configured to that the cutting member **6** is partially inside the casing **C** and partially outside thereof.

The casing **C** may comprise two opposite orifices **C1, C1'** for the passage of tube sections, the orifices **C1, C1'** are disposed in the support direction. The casing **C** may comprise two additional opposite orifices **C2, C2'** for the passage of rod or tube sections, the additional orifices **C2, C2'** are disposed in the additional direction.

### Examples of method

### Example 1

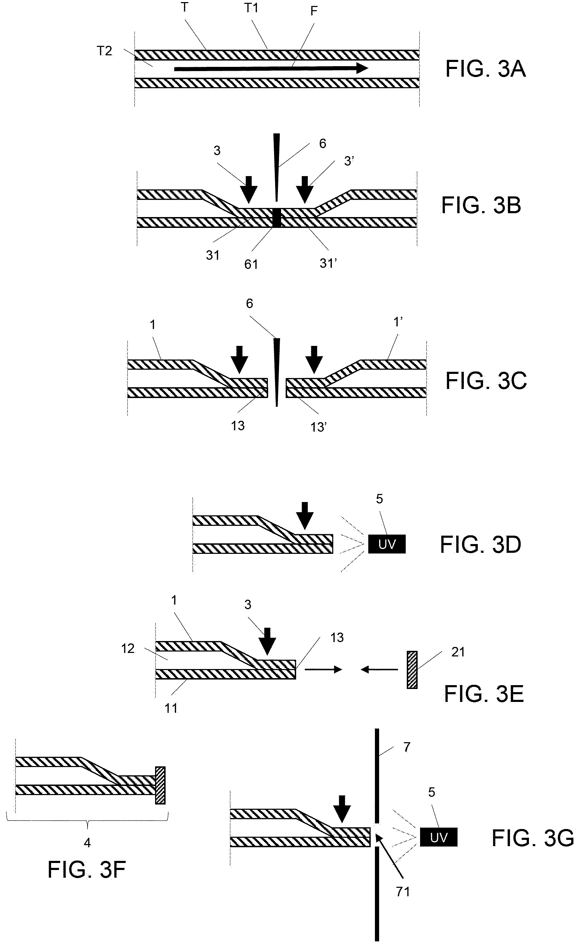

Example 1 of the method will be described hereafter with reference to Fig. **3A** to **3F****.** Example 1 is a sealing embodiment aiming at sealing a cut end of a silicone tube with a connecting element. The connecting element is a stop in the form of a slice of material **21** with a cross section contacting and covering a cross section of the cut end. This method can be carried out while fluid is flowing through the silicone tube.

In this example, the silicone tube with the cut end may be provided as such or the method may start with a silicone tube **T** with a continuous wall **T1** and a central lumen **T2** thereinside. The central lumen **T2** extends along a longitudinal axis and allows a fluid to flow therethrough. (See Fig. **3A****.)** The method then also comprises providing a cut end **13** to the silicone tube **T.**

Providing the cut end **13** may comprise cutting the silicone tube **T** to obtain two portions **1, 1'** of silicone tube, each with a cut end **13, 13'** respectively.

Cutting the silicone tube **T** may comprise sterilising a cutting member **6** (not shown) and transversely cutting a silicone tube **T** with the cutting member **6** (Fig. **3C****)** at a cutting site **61.** The cutting member **6** is preferably at room temperature during cutting.

Prior to cutting the silicone tube **T,** the method may comprise clamping the silicone tube **T** at a clamping site **31** and a further clamping site **31'** for example using two tube-flatteners **3, 3';** thereby providing a flattened zone between the clamping site **31** and the further clamping site **31',** otherwise called a flattened region or a sealed region (Fig. **3B****).**

The clamping site **31** and the further clamping site **31'** are chosen so that the cutting site **61** lies between them. The cutting site **61** is sufficiently close to the clamping sites **31, 31'** so that the cut ends **13, 13'** are in a clamped state (see Fig. **2A** or **2B****).**

The description is now focused on one portion **1** of the silicone tube, which will be simply called silicone tube **1** for the ease of description.

The method comprises sterilising the cut end **13** of the silicone tube **1** and the slice of material **21** using a UVC radiation **5** (Fig. **3D****)** thereby obtaining a sterile cut end **13** and a sterile slice of material **21.** The method further comprises connecting the cut end **13** of the silicone tube **1** with the slice of material **21** (Fig. 3E) thereby providing an adhesiveless connected assembly **4** (see Fig. **3F****).**

Sterilising the cut end and the slice of material may be carried out through UVC radiation **5,** preferably VUV radiation. The cut end **13** and a slice of material **21** might undergo sterilisation through UVC radiation concomitantly.

Connecting further comprises contacting the sterile slice of material with the sterile cut end (Fig. **3E****),** thereby obtaining an adhesiveless connected assembly **4** (Fig. **3F****).**

### Example 2

Example 2 of the method will be described hereafter with reference to Fig. **3G****.** Example 2 is mostly identical to Example 1 with the sole difference that sterilising the cut end is performed through a UVC-protection screen **7** with a cut out **71.** The UVC-protection screen **7** is positioned so that the cut out **71** faces the cut end.

### Example 3

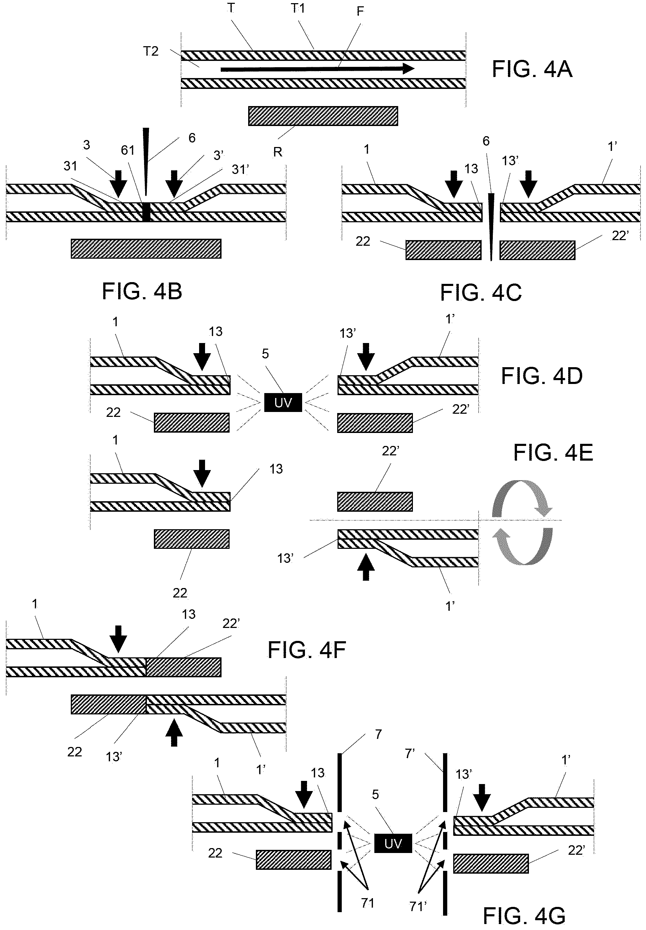

Example 3 of the method will be described hereafter with reference to Fig. **4A** to **4F** Example 3 is a sealing embodiment wherein the connecting element is a stop in the form of a rod **R** with a cross section contacting and covering a cross section of the cut end. This method can be carried out while fluid **F** is flowing through the silicone tube **T.**

In this example, the silicone tube with the cut end and the rod may be provided as such. Alternatively, they are provided through the following steps illustrated by Fig. **4A** to **4C****.**

The method may thus start with a silicone tube **T** with a continuous wall **T1** and a central lumen **T2** thereinside, and with a rod **R.** The central lumen **T2** extends along a longitudinal axis and allows a fluid to flow therethrough. (See Fig. **4A****.)** The method then also comprises providing a cut end **13** to the silicone tube **T.**

Providing the cut end **13** may comprise disposing the silicone tube and the rod **R** so that their longitudinal axes are colinear with each other (Fig. **4A****).**

Providing the cut end **13** may also comprise clamping the silicone tube **T** at a clamping site **31** and a further clamping site **31',** for example using tube-flatteners **3 and 3',** thereby providing a flattened zone between the clamping site **31** and the further clamping site **31',** otherwise called a flattened region or a sealed region (Fig. **4B****).**

Disposing and clamping can be carried in any order.

Providing the cut end **13** may also comprise cutting the silicone tube **T** and the rod **R** to obtain two portions **1, 1'** of silicone tube, each with a cut end **13, 13'** respectively, and two portions **2, 2'** of rod (Fig. **4C****).**

Cutting may comprise sterilising a cutting member **6** such as a cutting blade and transversely cutting the silicone tube **T** and the rod **R** with the cutting member at a cutting site **61** between the clamping site **31** and the further clamping site **31',** thereby providing two sets, one set on each side of the cutting site. Each set is made of a portion **1, 1'** of the silicone tube having a cut end **13, 13',** and a portion **22, 22'** the rod also having a cut end. The cutting site **61** is sufficiently close to the clamping site **31** and further clamping site **31'** so that the cut ends **13, 13'** of the silicone tube are in a clamped state.

The method comprises sterilising the cut end **13** of the silicone tube **1** and the rod **R** using a UVC radiation **5** (Fig. **4D****)** and connecting it with the rod **R** thereby providing an adhesiveless connected assembly **4** (see Fig. **4F****).** As shown hereafter, this method actually provides two connected assemblies.

Sterilising each cut ends **13, 13'** of the portions of the silicone tube **1, 1'** and portions of the rod **22, 22'** is preferably carried out with a VUV radiation **5,** thereby obtaining sterile tube cut ends and sterile rod cut ends.

Connecting may further comprise rotating one set relatively to the other set by 180° and around a rotation axis which is colinear with the longitudinal axes of the tube portions **1, 1'** and the rod portions **(22, 22'),** thereby positioning the cut end of a portion of the UV-transparent silicone tube **(13, 13')** in one set facing the cut end of a portion of the rod in the other set (Fig. **4E****).**

Rotating and sterilising may be carried out in any order.

Connecting further comprises contacting a sterile tube cut end of **13, 13'** one set with the sterile rod cut end of the other set, thereby forming two connected assemblies **4, 4'** of a silicone tube **1** and rod **22'.**

### Example 4

Example 4 of the method will be described hereafter with reference to Fig. **4G****.** Example 4 is mostly identical to Example 3 with the sole difference that sterilising the cut ends is performed through a UVC-protection screen **7, 7'** with cut outs **71, 71'.** The UVC-protection screens **7, 7'** are positioned so that the cut outs face the cut ends.

### Example 5

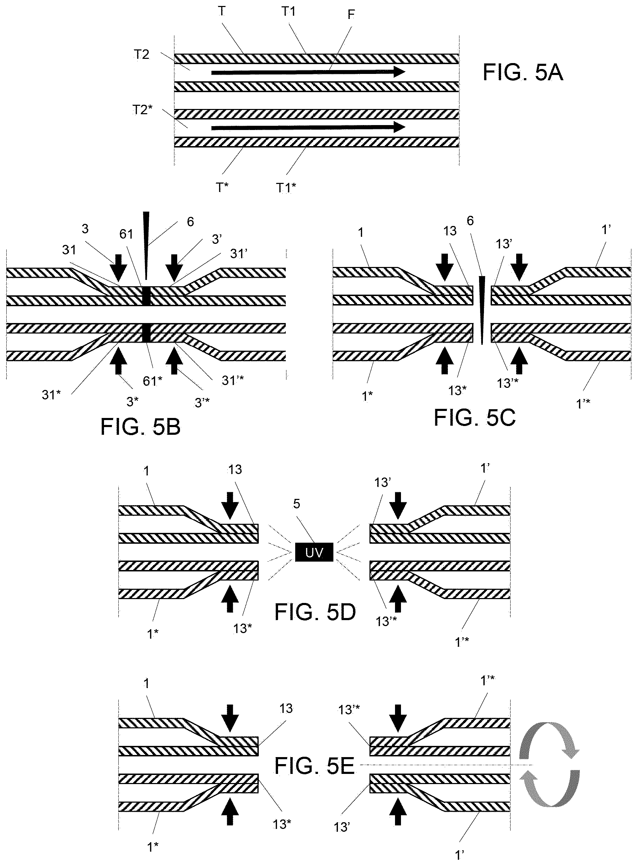

Example 5 of the method will be described hereafter with reference to Fig. **5A** to **5G** Example 5 is a welding embodiment wherein the connecting element is another silicone tube **T*.** This method can be carried out while fluid is flowing through the silicone tubes **(T, T*),** through the central lumen thereof **(T2, T2*)** serving as fluid pathways.

The silicone tubes **T** and **T*** are chosen to have same cross sections.

In this example, both silicones tubes with cut ends may be provided as such. Alternatively, they are provided through the following steps illustrated by Fig. **5A** to **5C****.**

The method may thus start with two silicone tubes **T, T*,** each with a continuous wall **T1, T1*** and a central lumen **T2, T2*** thereinside. The central lumen **T2, T2*** extends along a longitudinal axis and allows a fluid to flow therethrough. (See Fig. **5A****.)** The method then also comprises providing a cut end **13, 13*** to the silicone tubes **T, T*.**

Providing the cut ends **13, 13*** may comprise disposing the silicone tubes **T, T*** so that their longitudinal axes are colinear with each other (Fig. **5A****).**

Providing the cut end **13** may also comprise clamping the silicone tubes **T** at a clamping site **31** and a further clamping site **31',** for example using tube-flatteners **3** and **3',** thereby providing a flattened zone between the clamping site **31** and the further clamping site **31',** otherwise called a flattened region or a sealed region. Providing the cut end **13*** may also comprise clamping the silicone tubes **T*** at a clamping site **31*** and a further clamping site **31'*,** for example using the same tube-flatteners **3 and 3',** thereby providing a flattened zone between the clamping site **31*** and the further clamping site **31'*.** (See Fig. **5B****.)**

Disposing and clamping can be carried in any order.

Providing the cut ends **13, 13*** may also comprise cutting the silicone tubes **T, T*** to obtain four portions **1, 1', 1*, 1'*** of silicone tube, each with a cut end **13, 13', 13*, 13'*** respectively (Fig. **5C****).**

Cutting may comprise sterilising a cutting member **6** such as a cutting blade and transversely cutting the silicone tubes **T, T*** with the cutting member **6** at a cutting site **61** between the clamping sites **31, 31*** and the further clamping sites **31', 31'*,** thereby providing two sets, one set on each side of the cutting site **61.** Each set is made of a portion **1, 1'** of the silicone tube **T** having a cut end **13, 13',** and a portion **1*, 1'*** of the further silicone tube **T*** also having a cut end **13*, 13'*.** The cutting site **61** is sufficiently close to the clamping sites **31, 31*** and further clamping sites **31', 31'*** so that the cut ends **13, 13', 13*, 13'*** of the silicone tubes **T, T*** are in a clamped state.

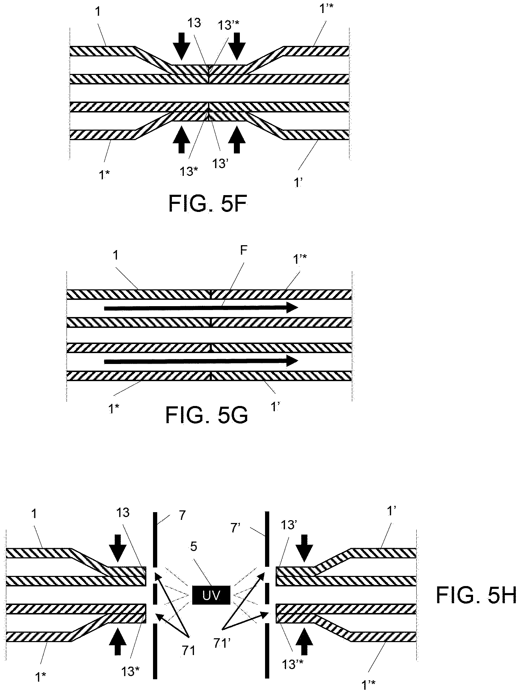

The method comprises sterilising the cut ends **13, 13'** of the silicone tube T and the cut ends **13*, 13'*** of the second silicone tube **T*** using a UVC radiation **5** (Fig. **5D****);** and connecting the cut end **13, 13'** of one silicone tube with the cut end **13*, 13*'** of another silicone tube (Fig. **5F****);** thereby providing an adhesiveless connected assembly. As shown hereafter, this method actually provides two connected assemblies.

Sterilising each cut ends **13, 13', 13*, 13'* of** the portions of the silicone tubes **T, T*** is preferably carried out with a VUV radiation **5,** thereby obtaining sterile tube cut ends.

Connecting may further comprise rotating one set relatively to the other set by 180° and around a rotation axis which is colinear with the longitudinal axes of the tube portions, thereby positioning the cut end of a portion of the first silicone tube in one set facing the cut end of a portion of the second silicone tube in the other set (Fig. **5E****).**

Rotating and sterilising may be carried out in any order.

Connecting further comprises contacting a sterile tube cut end of one set with the sterile tube cut end of the other set, thereby forming two connected assemblies of portions of silicone tubes (Fig. **5F****).**

The method may further comprise unclamping the clamping sites **31, 31* to** allow the cut end to reopen and return to an unclamped open state (Fig. **5G****).** The fluid F, if any, that was previously prevented from passing through the lumens **T2, T2*** of the tubes may now flow therethrough.

### Example 6

Example 6 of the method will be described hereafter with reference to Fig. **5H****.** Example 6 is mostly identical to Example 5 with the sole difference that sterilising the cut ends **13, 13*, 13', 13'*** of the portions of the silicone tubes **1, 1*, 1', 1'*** is performed through UVC-protection screens **7, 7'** with cut outs **71, 71'.** The UVC-protection screens **7, 7'** are positioned so that the cut outs face the cut ends.

### Example 7

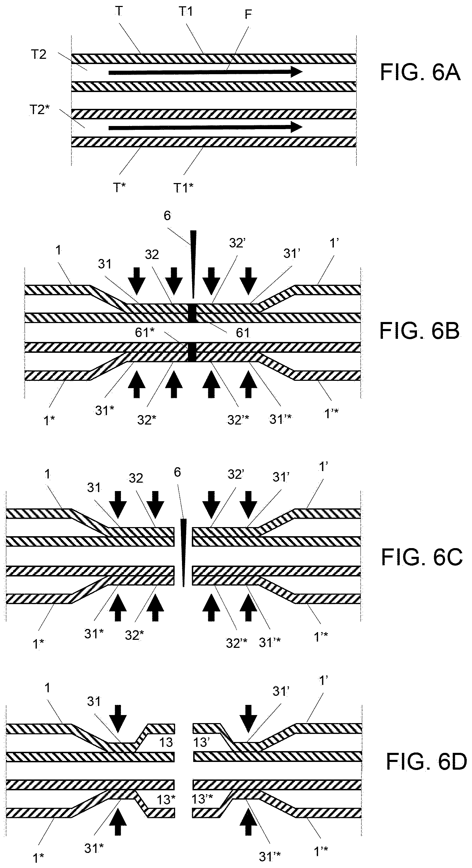

Example 7 of the method will be described hereafter with reference to Fig. **6A** to **6H****.** Example 7 is a welding embodiment wherein the connecting element is a heat shrinkable sleeve **8.** This method can be carried out while fluids are flowing through the silicone tubes **T, T*.**

In this example, two silicones tubes **T, T*** with cut ends **13, 13*** may be provided as such. Alternatively, they are provided through the following steps illustrated by Fig. **6A** to **6C****.**

The method may thus start with two silicone tubes **T, T*,** each with a continuous wall **T1, T1*** and a central lumen **T2, T2*** thereinside. The central lumen **T2, T2*** extends along a longitudinal axis and allows a fluid to flow therethrough. (See Fig. **6A****.)** The method then also comprises providing a cut end **13, 13*** to the silicone tubes **T, T*.**

Providing the cut ends **13, 13*** may comprise disposing the silicone tubes **T, T*** so that their longitudinal axes are colinear with each other (Fig. **6A****).**

Providing the cut end **13** may also comprise clamping the silicone tubes **T** at a clamping site **31,** a second clamping site **32,** a second further clamping site **32',** and a further clamping site **31'** in this order, for example using tube-flatteners **3** and **3',** thereby providing a flattened zone extending through the clamping site **31,** the second clamping site **32,** the second further clamping site **32'** and the further clamping site **31',** otherwise called a flattened region or a sealed region. Providing the cut end **13*** may also comprise clamping the silicone tubes **T*** at a clamping site **31*,** a second clamping site **32*,** a second further clamping site **32'*** and a further clamping site **31'*** in this order, for example using the same tube-flatteners **3 and 3',** thereby providing a flattened zone extending through the clamping site **31*,** the second clamping site **32*,** the second further clamping site **32'*** and the further clamping site **31'*.** (See Fig. **6B****.)**

Disposing and clamping can be carried in any order.

Providing the cut ends **13, 13*** may also comprise cutting the silicone tubes **T, T*** to obtain four portions **1, 1', 1*, 1'*** of silicone tube, each with a cut end **13, 13', 13*, 13'*** respectively (Fig. **6C****).**

Cutting may comprise sterilising a cutting member **6** such as a cutting blade and transversely cutting the silicone tubes **T, T*** with the cutting member **6** at a cutting site **61** between the second clamping sites **32, 32*** and the second further clamping sites **32', 32'*,** thereby providing two sets, one set on each side of the cutting site **61.** Each set is made of a portion **1, 1'** of the silicone tube **T** having a cut end **13, 13',** and a portion **1*, 1'*** of the further silicone tube **T*** also having a cut end **13*, 13'*.** The cutting site **61** is sufficiently close to the second clamping sites **32, 32*** and second further clamping sites **32', 32'*** so that the cut ends **13, 13', 13*, 13'*** of the silicone tubes **T, T*** are in a clamped state when clamped at these site, but sufficiently far away from the clamping site **31, 31*** and further clamping sites **31', 31'*** so that the cut ends **13, 13', 13*, 13'*** of the silicone tubes **T, T*** are in an unclamped state when no longer clamped at the second clamping sites **32, 32*** and second further clamping sites **32', 32'*.**

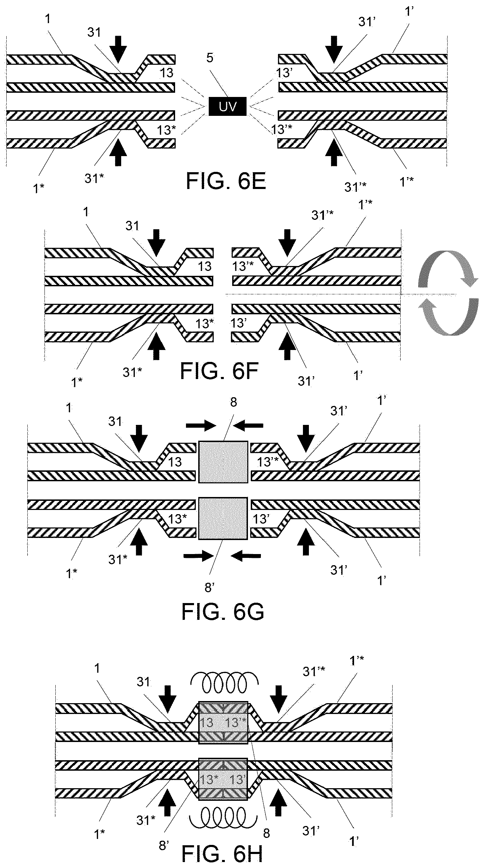

The method comprises sterilising the cut ends **13, 13', 13*, 13'*** using a UVC radiation (Fig. **6E****)** and connecting the cut end **13, 13', 13*, 13*'** through a heat shrinkable sleeve **7, 7'** (Fig. **6G** and **6H****);** thereby providing an adhesiveless connected assembly.

Sterilising the cut ends **13, 13', 13*, 13'*** may be preferably carried out using a VUV radiation.

Sterilising may further comprise unclamping the second clamping sites **32, 32*** and the second further clamping sites **32', 32'*,** notably before radiating the cut ends with the UVC radiation (Fig. **6D****).** The cutting site is sufficiently far from the first clamping sites and the first further clamping site so that the cut ends **13, 13*, 13', 13'*** of the silicone tubes **1, 1*, 1', 1'*** are in an unclamped state following this operation thus resulting in open cut ends.

Connecting may comprise rotating one set relatively to the other set by 180° and around a rotation axis which is colinear with the longitudinal axes of the tube portions, thereby positioning the cut end of a portion of the first silicone tube in one set facing the cut end of a portion of the second silicone tube in the other set (Fig. **6F****).**

Rotating and sterilising may be carried out in any order.

Connecting further comprises introducing the cut end **13** of one portion **1** the first silicone tube **T** in one set through one end of a first heat shrinkable sleeve **8,** introducing a cut end **13'*** of one portion **1'*** the second silicone tube **T*** in the other set through the other end of the first heat shrinkable sleeve **8,** introducing the cut end **13*** of the other portion **1*** the second silicone tube **T*** in the one set through one end of a second heat shrinkable sleeve **8',** and introducing a cut end **13'** of the other portion **1'** of the first silicone tube **T** in the other set through the other end of the second heat shrinkable sleeve **8'** (Fig. **6G****).**

Connecting further comprises heating the first and second heat shrinkable sleeves **8, 8',** thereby providing an adhesiveless seal between the cut ends **13, 13'*** of the silicone tubes **T, T*** forming a newly sealed tube and between the cut ends **13', 13*** the silicone tubes **T, T*** forming a second newly sealed tube (Fig. **6H****).**

The method may comprise unclamping the remaining clamped regions **(31, 31*, 31', 31'*)** to allow the lumen of the newly sealed tubes to reopen. The fluid that was previously prevented from flowing through the lumens of the silicone tubes may now flow therethrough.

### Example 8

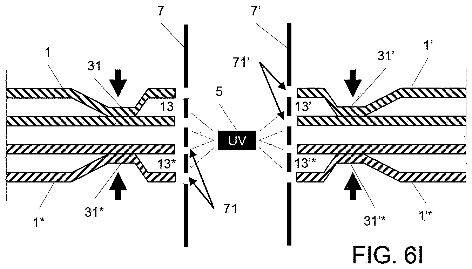

Example 8 is mostly identical to Example 7 with the difference that it comprises sterilising the cut ends and in particular through a UVC-protection screen **7, 7'** with cut outs **71, 71'** (Fig. 6I). The UV-protection screens **7, 7'** are positioned so that the cut outs face the open cut ends.

### Example 9

Example 9 (not illustrate) is mostly identical to Example 7 with the difference that the silicone tubes **T, T*** are clamped only at the clamping site **31, 31*** and the further clamping site **31', 31'*.** Then, the unclamped state of the cut ends **13, 13', 13*, 13'*** is obtained through moving the clamping site **31, 31*** and the further clamping site **31', 31'*** further away from the cut ends **13, 13', 13*, 13'*,** thus allowing these latter to regain an unclamped state. Example 9 is of course compatible with the features described in Example 8.

### Example of a device

Referring to Fig. **7A****,** the device 50 is configured for making a connection between a portion of a silicone tube **T** and a portion of a second silicone tube **T*** through one cut end of each. Such device is illustrated as including a casing **C** forming an operation chamber **CH** and having orifices **C1, C1', C2, C2'** for the passage of tubes **T, T*;** and inside the casing **C** a VUV radiation source **5** capable of sterilising and activating the cut ends **13, 13', 13*, 13'*,** a cutting member **6** and two sets of tube supports **TH1, TH1'** and **TH2, TH2'.**

The cutting member **6** is controlled between a rest position (as depicted in Fig. 7A) and a cutting position (not illustrated).

The device **50** also comprises a UVC radiation source **5'** capable of emitting UVC radiation to sterilise the cutting member **6.** In some cases, the UVC radiation source **5'** may be powerful enough to sterilise all surfaces reachable by the radiation.

The device **50** also comprises a driver **D** to allow movement of tube supports **TH1, TH1', TH2, TH2'.**

Fig. **7B** schematically shows the lower part of the device **50** after cutting has been performed. Here, the tube sections **1, 1',** and the tube sections **1*, 1'*** have been pulled apart from each other by the driver **D** so they lie on both side of the VUV lamp **5.** Also, the driver **D** has rotated a set of tube support **TH1** and tube support **TH2** by 180° about an axis **A** which is colinear with the longitudinal axes of the tube sections **1, 1', 1*,** 1'*Fig. **7C** schematically shows the outcome of the connection of the tube sections **1, 1', 1*, 1'*** following rotation. Here, the driver **D** has brought tube support **TH1** closer to the tube support **TH2'** and tube support **TH1'** closer to tube support **TH2** so that the cut ends of the corresponding tube sections are contacting each other and can be joined.

Also, the environment **55** within the operation chamber **CH** can be controlled by environment controller **W as** illustrated in Fig. **7C****.**

## Claims

1. A method for sterilely connecting a cut end (13, 13') of a silicone tube (T) with a connecting element (2, 21, R, T*), the method comprising:
- sterilising the cross-section of the cut end using a UVC radiation (5) thereby obtaining a sterilised cut end; and
- connecting the cut end with the connecting element thereby providing an adhesiveless connected assembly (4), wherein the UVC radiation comprises at least partially VUV radiation;
the method further comprising before sterilising the cut end and sterilising the connecting element: clamping the silicone tube at a clamping site (31, 31') thereof, thereby obtaining a sealed region within the tube;
transversely cutting the silicone tube at a cutting site (61) adjacent the clamping site, thereby providing the cut end.

2. The method of claim 1, further comprising sterilising at least one of:
- the connecting element using the UVC radiation thereby obtaining a sterilised connecting element; and
- the environment of the silicone tube and connecting element.

3. The method of claim 1 or 2, wherein it is carried out in a controlled environment.

4. The method of any one of the previous claims, wherein sterilising the cut end and sterilising the connecting element are performed concomitantly.

5. The method of any one of the previous claims, wherein the connecting element is a slice of material (21) with a cross section contacting and covering the cross section of the sterilised cut end to form a stop.

6. The method of any one of claims 1 to 5, wherein the cut end has a cross section, and wherein the connecting element is a rod (R) with a cross section contacting or covering the cross section of the cut end to form a stop.

7. The method of claim 6, comprising:
- clamping the silicone tube at a clamping site (31) thereof and a further clamping site (31') thereof, the cutting site being positioned between the clamping site and the further clamping site;
- transversely cutting the silicone tube at a cutting site between the clamping site and the further clamping site using a cutting member (6), thereby providing two tube portions (1, 1') each having a cut end (13, 13');
- transversely cutting the rod using the cutting member, thereby providing two rod portions (22, 22'), each having a rod cut end; and
- contacting each rod cut end with a corresponding tube cut end.

8. The method of claim 7, wherein each of the tube and rod has a longitudinal axis, wherein the method further comprises:
- before transversely cutting the tube, disposing the tube and the rod so that their longitudinal axes are colinear with each other, thereby transversely cutting provides two sets, each having a tube portion and a rod portion; and
- rotating one set relatively to the other set by 180° and around a rotation axis which is colinear with the longitudinal axes of the tube portions and the rod portions;
wherein transversely cutting the tube and transversely cutting the rod are performed with a single relative movement of the cutting member.

9. The method of any one of claims 1 to 4, wherein the connecting element is a second silicone tube (T*); and wherein the method further comprises:
- clamping the silicone tube at a clamping site (31) thereof and a further clamping site (31') thereof, the cutting site being positioned between the clamping site and the further clamping site;
- transversely cutting the silicone tube at the cutting site adjacent the clamping site and the further clamping site using a cutting member (6), preferably unheated, thereby providing two tube portions (1, 1') each having a cut end (13, 13');
- clamping the second silicone tube at a clamping site (31*) and a further clamping site (31'*) thereof;
- transversely cutting the second tube at a cutting site between the clamping and the further clamping site, thereby obtaining two tube portions (1*, 1'*) each having a cut end (13*, 13'*); and
- contacting each cut end of the silicone tube with a corresponding cut end of the second silicone tube portions.

10. The method of claim 9, wherein each of the silicone tube and second silicone tube has a longitudinal axis, and wherein the method further comprises:
- before transversely cutting the silicone tube and the second silicone tube, disposing the silicone tube and the second silicone tube so that their longitudinal axes are colinear with each other, thereby transversely cutting provides two sets, each having a tube portion of the silicone tube and a tube portion of the second silicone tube; and
- rotating one set relatively to the other set by 180° and around a rotation axis which is colinear with the longitudinal axes of the tube portions;
wherein transversely cutting the silicone tube and transversely cutting the second silicone tube are performed with a single relative movement of the cutting member.

11. The method of any one of claims 1 to 4, wherein the connecting element is a heat shrinkable sleeve (8, 8') with two open ends, wherein connecting comprises introducing the sterilised cut end through one end of the heat shrinkable sleeve, introducing a sterilised cut end (13'*) of a second silicone tube (T*) through the other end of the heat shrinkable sleeve and heating the heat shrinkable sleeve, thereby providing an adhesiveless seal at the cut ends of the silicone tube and the second silicone tube.

12. A device (50) comprising an operation chamber (CH), and within the operation chamber:
- a tube support (TH1, TH1') configured to receive a silicone tube (T);
- an additional support (TH2, TH2') configured to receive a connecting element (2, 21, R, T*);
- a UVC radiation source (5) configured to sterilise the silicone tube received on the tube support; and
- a driver (R) configured to move the tube support and the additional support relative to each other, wherein the UVC radiation comprises at least partially VUV radiation.

13. The device of claim 12, further comprising a movable cutting member (6) configured to move from a rest position to a cutting position and along a cutting direction in order to cut the silicone tube received on the tube support.

## Patentansprüche

1. Verfahren zum sterilen Verbinden eines Schnittendes (13, 13') eines Silikonschlauchs (T) mit einem Verbindungselement (2, 21, R, T*), wobei das Verfahren umfasst:
- Sterilisieren des Querschnitts des Schnittendes unter Verwendung einer UVC-Strahlung (5), um dadurch ein sterilisiertes Schnittende zu erhalten; und
- Verbinden des Schnittendes mit dem Verbindungselement, um dadurch eine klebstofffreie verbundene Anordnung (4) bereitzustellen, wobei die UVC-Strahlung zumindest teilweise VUV-Strahlung umfasst;
wobei das Verfahren vor dem Sterilisieren des Schnittendes und dem Sterilisieren des Verbindungselements ferner umfasst:
Abklemmen des Silikonschlauchs an einer Klemmstelle (31, 31') desselben, um dadurch einen abgedichteten Bereich innerhalb des Schlauchs zu erhalten;
Querdurchtrennen des Silikonschlauchs an einer Schneidstelle (61) angrenzend an die Klemmstelle, um dadurch das Schnittende bereitzustellen.

2. Verfahren nach Anspruch 1, ferner umfassend das Sterilisieren mindestens eines von:
- dem Verbindungselement unter Verwendung der UVC-Strahlung, um dadurch ein sterilisiertes Verbindungselement zu erhalten; und
- der Umgebung des Silikonschlauchs und des Verbindungselements.

3. Verfahren nach Anspruch 1 oder 2, wobei es in einer kontrollierten Umgebung durchgeführt wird.

4. Verfahren nach einem der vorhergehenden Ansprüche, wobei das Sterilisieren des Schnittendes und das Sterilisieren des Verbindungselements gleichzeitig durchgeführt werden.

5. Verfahren nach einem der vorhergehenden Ansprüche, wobei das Verbindungselement eine Materialscheibe (21) mit einem Querschnitt ist, der den Querschnitt des sterilisierten Schnittendes berührt und bedeckt, um einen Stopfen zu bilden.

6. Verfahren nach einem der Ansprüche 1 bis 5, wobei das Schnittende einen Querschnitt aufweist und wobei das Verbindungselement ein Stab (R) mit einem Querschnitt ist, der den Querschnitt des Schnittendes berührt oder bedeckt, um einen Stopfen zu bilden.

7. Verfahren nach Anspruch 6, umfassend:
- Abklemmen des Silikonschlauchs an einer Klemmstelle (31) desselben und einer weiteren Klemmstelle (31') desselben, wobei die Schneidstelle zwischen der Klemmstelle und der weiteren Klemmstelle positioniert ist;
- Querdurchtrennen des Silikonschlauchs an einer Schneidstelle zwischen der Klemmstelle und der weiteren Klemmstelle unter Verwendung eines Schneidelements (6), um dadurch zwei Schlauchabschnitte (1, 1') bereitzustellen, die jeweils ein Schnittende (13, 13') aufweisen;
- Querdurchtrennen des Stabs unter Verwendung des Schneidelements, um dadurch zwei Stababschnitte (22, 22') bereitzustellen, die jeweils ein Stab-Schnittende aufweisen; und
- Inkontaktbringen jedes Stab-Schnittendes mit einem entsprechenden Schlauch-Schnittende.

8. Verfahren nach Anspruch 7, wobei jeder von dem Schlauch und dem Stab eine Längsachse aufweist, wobei das Verfahren ferner umfasst:
- vor dem Querdurchtrennen des Schlauchs, Anordnen des Schlauchs und des Stabs derart, dass ihre Längsachsen kollinear zueinander sind, so dass das Querdurchtrennen zwei Sätze bereitstellt, die jeweils einen Schlauchabschnitt und einen Stababschnitt aufweisen; und
- Drehen eines Satzes relativ zum anderen Satz um 180° und um eine Drehachse, die kollinear mit den Längsachsen der Schlauchabschnitte und der Stababschnitte ist;
wobei das Querdurchtrennen des Schlauchs und das Querdurchtrennen des Stabs mit einer einzigen Relativbewegung des Schneidelements durchgeführt werden.

9. Verfahren nach einem der Ansprüche 1 bis 4, wobei das Verbindungselement ein zweiter Silikonschlauch (T*) ist; und wobei das Verfahren ferner umfasst:
- Abklemmen des Silikonschlauchs an einer Klemmstelle (31) desselben und einer weiteren Klemmstelle (31') desselben, wobei die Schneidstelle zwischen der Klemmstelle und der weiteren Klemmstelle positioniert ist;
- Querdurchtrennen des Silikonschlauchs an der Schneidstelle angrenzend an die Klemmstelle und die weitere Klemmstelle unter Verwendung eines Schneidelements (6), vorzugsweise unbeheizt, um dadurch zwei Schlauchabschnitte (1, 1') bereitzustellen, die jeweils ein Schnittende (13, 13') aufweisen;
- Abklemmen des zweiten Silikonschlauchs an einer Klemmstelle (31*) und einer weiteren Klemmstelle (31'*) desselben;
- Querdurchtrennen des zweiten Schlauchs an einer Schneidstelle zwischen der Klemmung und der weiteren Klemmstelle, um dadurch zwei Schlauchabschnitte (1*, 1'*) zu erhalten, die jeweils ein Schnittende (13*, 13'*) aufweisen; und
- Inkontaktbringen jedes Schnittendes des Silikonschlauchs mit einem entsprechenden Schnittende der zweiten Silikonschlauchabschnitte.

10. Verfahren nach Anspruch 9, wobei jeder von dem Silikonschlauch und dem zweiten Silikonschlauch eine Längsachse aufweist, und wobei das Verfahren ferner umfasst:
- vor dem Querdurchtrennen des Silikonschlauchs und des zweiten Silikonschlauchs, Anordnen des Silikonschlauchs und des zweiten Silikonschlauchs derart, dass ihre Längsachsen kollinear zueinander sind, so dass das Querdurchtrennen zwei Sätze bereitstellt, die jeweils einen Schlauchabschnitt des Silikonschlauchs und einen Schlauchabschnitt des zweiten Silikonschlauchs aufweisen; und
- Drehen eines Satzes relativ zum anderen Satz um 180° und um eine Drehachse, die kollinear mit den Längsachsen der Schlauchabschnitte ist;
wobei das Querdurchtrennen des Silikonschlauchs und das Querdurchtrennen des zweiten Silikonschlauchs mit einer einzigen Relativbewegung des Schneidelements durchgeführt werden.

11. Verfahren nach einem der Ansprüche 1 bis 4, wobei das Verbindungselement eine wärmeschrumpfbare Hülse (8, 8') mit zwei offenen Enden ist, wobei das Verbinden das Einführen des sterilisierten Schnittendes durch ein Ende der wärmeschrumpfbaren Hülse, das Einführen eines sterilisierten Schnittendes (13'*) eines zweiten Silikonschlauchs (T*) durch das andere Ende der wärmeschrumpfbaren Hülse und das Erwärmen der wärmeschrumpfbaren Hülse umfasst, um dadurch eine klebstofffreie Abdichtung an den Schnittenden des Silikonschlauchs und des zweiten Silikonschlauchs bereitzustellen.

12. Vorrichtung (50), umfassend eine Arbeitskammer (CH) und innerhalb der Arbeitskammer:
- eine Schlauchhalterung (TH1, TH1'), die dazu ausgebildet ist, einen Silikonschlauch (T) aufzunehmen;
- eine zusätzliche Halterung (TH2, TH2'), die dazu ausgebildet ist, ein Verbindungselement (2, 21, R, T*) aufzunehmen;
- eine UVC-Strahlungsquelle (5), die dazu ausgebildet ist, den auf der Schlauchhalterung aufgenommenen Silikonschlauch zu sterilisieren; und
- einen Antrieb (R), der dazu ausgebildet ist, die Schlauchhalterung und die zusätzliche Halterung relativ zueinander zu bewegen, wobei die UVC-Strahlung zumindest teilweise VUV-Strahlung umfasst.

13. Vorrichtung nach Anspruch 12, ferner umfassend ein bewegliches Schneidelement (6), das dazu ausgebildet ist, sich von einer Ruhestellung in eine Schneidstellung und entlang einer Schneidrichtung zu bewegen, um den auf der Schlauchhalterung aufgenommenen Silikonschlauch zu schneiden.

## Revendications

1. Procédé pour raccorder de manière stérile une extrémité coupée (13, 13') d'un tube en silicone (T) à un élément de raccordement (2, 21, R, T*), le procédé comprenant :
- la stérilisation de la section transversale de l'extrémité coupée à l'aide d'un rayonnement UVC (5), obtenant ainsi une extrémité coupée stérilisée ; et
- le raccordement de l'extrémité coupée à l'élément de raccordement, fournissant ainsi un ensemble raccordé sans adhésif (4), dans lequel le rayonnement UVC comprend au moins partiellement un rayonnement VUV ;
le procédé comprenant en outre, avant la stérilisation de l'extrémité coupée et la stérilisation de l'élément de raccordement :
le pincement du tube en silicone au niveau d'un site de pincement (31, 31') de celui-ci, obtenant ainsi une région scellée à l'intérieur du tube ;
la coupe transversale du tube en silicone au niveau d'un site de coupe (61) adjacent au site de pincement, fournissant ainsi l'extrémité coupée.

2. Procédé selon la revendication 1, comprenant en outre la stérilisation d'au moins l'un parmi :
- l'élément de raccordement à l'aide du rayonnement UVC, obtenant ainsi un élément de raccordement stérilisé ; et
- l'environnement du tube en silicone et de l'élément de raccordement.

3. Procédé selon la revendication 1 ou 2, dans lequel il est réalisé dans un environnement contrôlé.

4. Procédé selon l'une quelconque des revendications précédentes, dans lequel la stérilisation de l'extrémité coupée et la stérilisation de l'élément de raccordement sont réalisées de manière concomitante.

5. Procédé selon l'une quelconque des revendications précédentes, dans lequel l'élément de raccordement est une tranche de matériau (21) présentant une section transversale en contact avec et recouvrant la section transversale de l'extrémité coupée stérilisée pour former un bouchon.

6. Procédé selon l'une quelconque des revendications 1 à 5, dans lequel l'extrémité coupée présente une section transversale, et dans lequel l'élément de raccordement est une tige (R) présentant une section transversale en contact avec ou recouvrant la section transversale de l'extrémité coupée pour former un bouchon.

7. Procédé selon la revendication 6, comprenant :
- le pincement du tube en silicone au niveau d'un site de pincement (31) de celui-ci et d'un site de pincement supplémentaire (31') de celui-ci, le site de coupe étant positionné entre le site de pincement et le site de pincement supplémentaire ;
- la coupe transversale du tube en silicone au niveau d'un site de coupe entre le site de pincement et le site de pincement supplémentaire à l'aide d'un élément de coupe (6), fournissant ainsi deux portions de tube (1, 1') ayant chacune une extrémité coupée (13, 13') ;
- la coupe transversale de la tige à l'aide de l'élément de coupe, fournissant ainsi deux portions de tige (22, 22') ayant chacune une extrémité coupée de tige ; et
- la mise en contact de chaque extrémité coupée de tige avec une extrémité coupée de tube correspondante.

8. Procédé selon la revendication 7, dans lequel chacun du tube et de la tige présente un axe longitudinal, dans lequel le procédé comprend en outre :
- avant la coupe transversale du tube, la disposition du tube et de la tige de sorte que leurs axes longitudinaux soient colinéaires l'un avec l'autre, de sorte que la coupe transversale fournit deux ensembles ayant chacun une portion de tube et une portion de tige ; et
- la rotation d'un ensemble par rapport à l'autre ensemble de 180° et autour d'un axe de rotation qui est colinéaire avec les axes longitudinaux des portions de tube et des portions de tige ;
dans lequel la coupe transversale du tube et la coupe transversale de la tige sont réalisées avec un seul mouvement relatif de l'élément de coupe.

9. Procédé selon l'une quelconque des revendications 1 à 4, dans lequel l'élément de raccordement est un deuxième tube en silicone (T*) ; et dans lequel le procédé comprend en outre :
- le pincement du tube en silicone au niveau d'un site de pincement (31) de celui-ci et d'un site de pincement supplémentaire (31') de celui-ci, le site de coupe étant positionné entre le site de pincement et le site de pincement supplémentaire ;
- la coupe transversale du tube en silicone au niveau du site de coupe adjacent au site de pincement et au site de pincement supplémentaire à l'aide d'un élément de coupe (6), de préférence non chauffé, fournissant ainsi deux portions de tube (1, 1') ayant chacune une extrémité coupée (13, 13') ;
- le pincement du deuxième tube en silicone au niveau d'un site de pincement (31*) et d'un site de pincement supplémentaire (31'*) de celui-ci ;
- la coupe transversale du deuxième tube au niveau d'un site de coupe entre le pincement et le site de pincement supplémentaire, obtenant ainsi deux portions de tube (1*, 1'*) ayant chacune une extrémité coupée (13*, 13'*) ; et
- la mise en contact de chaque extrémité coupée du tube en silicone avec une extrémité coupée correspondante des portions de deuxième tube en silicone.

10. Procédé selon la revendication 9, dans lequel chacun du tube en silicone et du deuxième tube en silicone présente un axe longitudinal, et dans lequel le procédé comprend en outre :
- avant la coupe transversale du tube en silicone et du deuxième tube en silicone, la disposition du tube en silicone et du deuxième tube en silicone de sorte que leurs axes longitudinaux soient colinéaires l'un avec l'autre, de sorte que la coupe transversale fournit deux ensembles ayant chacun une portion de tube du tube en silicone et une portion de tube du deuxième tube en silicone ; et
- la rotation d'un ensemble par rapport à l'autre ensemble de 180° et autour d'un axe de rotation qui est colinéaire avec les axes longitudinaux des portions de tube ;
dans lequel la coupe transversale du tube en silicone et la coupe transversale du deuxième tube en silicone sont réalisées avec un seul mouvement relatif de l'élément de coupe.

11. Procédé selon l'une quelconque des revendications 1 à 4, dans lequel l'élément de raccordement est un manchon thermorétractable (8, 8') présentant deux extrémités ouvertes, dans lequel le raccordement comprend l'introduction de l'extrémité coupée stérilisée à travers une extrémité du manchon thermorétractable, l'introduction d'une extrémité coupée stérilisée (13'*) d'un deuxième tube en silicone (T*) à travers l'autre extrémité du manchon thermorétractable et le chauffage du manchon thermorétractable, fournissant ainsi un joint sans adhésif au niveau des extrémités coupées du tube en silicone et du deuxième tube en silicone.

12. Dispositif (50) comprenant une chambre d'opération (CH), et à l'intérieur de la chambre d'opération :
- un support de tube (TH1, TH1') configuré pour recevoir un tube en silicone (T) ;
- un support additionnel (TH2, TH2') configuré pour recevoir un élément de raccordement (2, 21, R, T*) ;
- une source de rayonnement UVC (5) configurée pour stériliser le tube en silicone reçu sur le support de tube ; et
- un dispositif d'entraînement (R) configuré pour déplacer le support de tube et le support additionnel l'un par rapport à l'autre, dans lequel le rayonnement UVC comprend au moins partiellement un rayonnement VUV.

13. Dispositif selon la revendication 12, comprenant en outre un élément de coupe mobile (6) configuré pour se déplacer d'une position de repos à une position de coupe et selon une direction de coupe afin de couper le tube en silicone reçu sur le support de tube.
